Europäisches Patentamt

European Patent Office

Office européen des brevets

(19)

(11) Veröffentlichungsnummer: **0 237 973**
**A1**

(12) **EUROPÄISCHE PATENTANMELDUNG**

(21) Anmeldenummer: **87103641.4**

(22) Anmeldetag: **13.03.87**

(51) Int. Cl.³: **A 61 M 5/00**
A 61 M 1/14, A 61 J 1/00
B 65 D 85/00

(30) Priorität: **17.03.86 DE 8607304 U**

(43) Veröffentlichungstag der Anmeldung:
**23.09.87 Patentblatt 87/39**

(84) Benannte Vertragsstaaten:
**AT BE CH DE FR GB IT LI LU NL SE**

(71) Anmelder: **Saueressig, Ulrich, Dr. med.**
**Hofaue 91-93**
**D-5600 Wuppertal 1(DE)**

(72) Erfinder: **Saueressig, Ulrich, Dr. med.**
**Hofaue 91-93**
**D-5600 Wuppertal 1(DE)**

(74) Vertreter: **Patentanwälte Dipl.-Ing. Walter Kuborn**
**Dipl.-Phys. Dr. Peter Palgen**
**Mulvanystrasse 2**
**D-4000 Düsseldorf(DE)**

(54) **Kunststoffbeutel zur Bevorratung von Dialysekonzentrat.**

(57) Der Kunststoffbeutel (10) weist an seinem oberen Rand (3) sowohl mindestens eine Öse (6) zum Aufhängen als auch die Entnahme- und Einfüllöffnung (12) auf, die sich dadurch oberhalb des Füllniveaus (13) des in den Kunststoffbeutel (10) befindlichen Konzentrats (14) befindet. Hierdurch wird die Handhabung bei der Entnahme des Konzentrats (14) und beim Zudosieren von Regaenzien durch die Entnahme- und Einfüllöffnung (12) erleichtert.

FIG.1

Croydon Printing Company Ltd.

EP 0 237 973 A1

PATENTANWÄLTE
**DIPL.-ING. WALTER KUBORN**
**DIPL.-PHYS. DR. PETER PALGEN**
ZUGELASSEN BEIM EUROPÄISCHEN PATENTAMT
**4000 DÜSSELDORF**
MULVANYSTRASSE 2 · TELEFON 63 27 27
TELEGRAMME: KUPAL
KREISSPARKASSE DÜSSELDORF NR. 1 014 463
DEUTSCHE BANK AG. DÜSSELDORF 2 919 207
POSTSCHECK-KONTO: KÖLN 115 211-504

0237973

Dr. med. Ulrich  S a u e r e s s i g
in 5600  W u p p e r t a l  1


Kunststoffbeutel zur Bevorratung von
Dialysekonzentrat


Die Erfindung betrifft einen Kunststoffbeutel zur
Bevorratung von Dialysekonzentrat.

Bekannt sind zur Zeit zur Bevorratung von Dialysekonzentraten Kunststoffkanister oder Beutel. Die festen
Kanister haben auf der Oberseite die Entnahme- und Zufüll-öffnung, sind aber in der Entsorgung sperrig.

Die bekannten Konzentratbeutel haben eine Entnahmevorrichtung in Form einer Steckverbindung, die sich
im Betriebszustand (hängend) unterhalb des Konzentratniveaus befindet. Eine Ausführungsform eines solchen Konzentratbeutels ist aus dem nicht vorveröffentlichten
DE-GM 85 33 481 bekannt.

Hierbei treten zwei Probleme auf:

1. Die Adaption der Ansaugeinrichtung des Dialysegerätes ist meistens nicht möglich.

- 2 -

2. Beim An- und Abschließen der Ansaugeinrichtung
   tritt Konzentrat aus. Der Beutel kann auch auslaufen.

Der Erfindung liegt die Aufgabe zugrunde, die Nachteile der bisher bekannten Kunststoffkanister und Beutel
aufzuheben.

Die erfindungsgemäße Lösung dieser Aufgabe ist in
Anspruch 1 wiedergegeben.

Durch die Anordnung der Entnahme- und Einfüllöffnung in der Nähe der Öse verbleibt sie bei hängendem Beutel über dem Füllniveau. Mit einem solchen Beutel ist
ein sauberes Arbeiten möglich, weil kein Konzentrat austreten kann. Ein besonderer Vorteil liegt darin, daß
eine Zudosierung von Elektrolyten oder Glucose über die
oberhalb des Füllniveaus liegende Entnahme- und Einfüllöffnung sauber und einfach möglich ist. Der Kunststoffbeutel kann an der Entnahme- und Einfüllöffnung einen
Verschluß erhalten, wie er von den festen Kunststoffkanistern bekannt ist. Die Ansaugeinrichtung der Dialysegeräte kann wie bei diesen Kanistern benutzt werden. Es
passen alle Konzentratansaugsysteme, die auch bei Kanistern zum Einsatz kommen können. Nach dem Entleeren des
Konzentratbeutels kann dieser platzsparend entsorgt werden, weil der Beutel zusammenfaltbar ist.

Die bevorzugte Ausführungsform des Kunststoffbeutels ist in Anspruch 2 wiedergegeben.

Das Verstärkungsstück nach Anspruch 3 hat die Aufgabe, die Öse zu bilden, die ja sonst an dem relativ
dünnen und zum Ausreißen neigenden Material des Kunststoffbeutels nicht einfach auszubilden wäre. Das Verstärkungsstück verteilt die durch das nicht unerhebliche Gewicht des gefüllten Kunststoffbeutels auftretenden

- 3 -

Kräfte außerdem auf einen größeren Bereich der Wandung des Kunststoffbeutels.

Damit der obere Rand des Kunststoffbeutels beim Aufhängen im wesentlichen seine Form behält, kann gemäß Anspruch 4 eine Rippe vorgesehen sein, die sich längs des oberen Randes erstreckt.

In einer zweckmäßigen Ausführungsform ist das Verstärkungsstück gemäß Anspruch 5 als dreieckiges flaches Plättchen ausgebildet, welches eine obere Ecke des Kunststoffbeutels bildet.

Das Verstärkungsstück kann nur an einer Ecke vorgesehen sein. Der Kunststoffbeutel hängt dann schräg.

Es ist aber auch möglich, das Verstärkungsstück an beiden oberen Ecken des Kunststoffbeutels vorzusehen und diesen an zwei Ösen aufzuhängen, so daß er gerade hängt.

Um eine vollständige Entleerung zu erleichtern, kann hierbei die Gestaltung nach Anspruch 6 getroffen sein, wodurch sich der letzte Rest des Konzentrats in der durch die Schräge gebildeten untersten Ecke sammelt und fast ganz aufgebraucht werden kann.

Dem Kunststoffbeutel kann gemäß Anspruch 7 ein Stützbehälter zugeordnet sein.

In der Zeichnung sind Ausführungsbeispiele der Erfindung schematisch dargestellt.

Fig. 1   zeigt einen an zwei Ösen gerade hängenden Konzentratbeutel mit eingebrachter Konzentratansaugeinrichtung;

Fig. 2   zeigt einen an nur einer Höhe hängenden Konzentratbeutel, dem ein zusätzlicher Inhaltsstoff zugesetzt wird;

Fig. 3   zeigt einen Konzentratbeutel, der vorübergehend in einem Stützbehälter aufrecht gehalten wird.

- 4 -

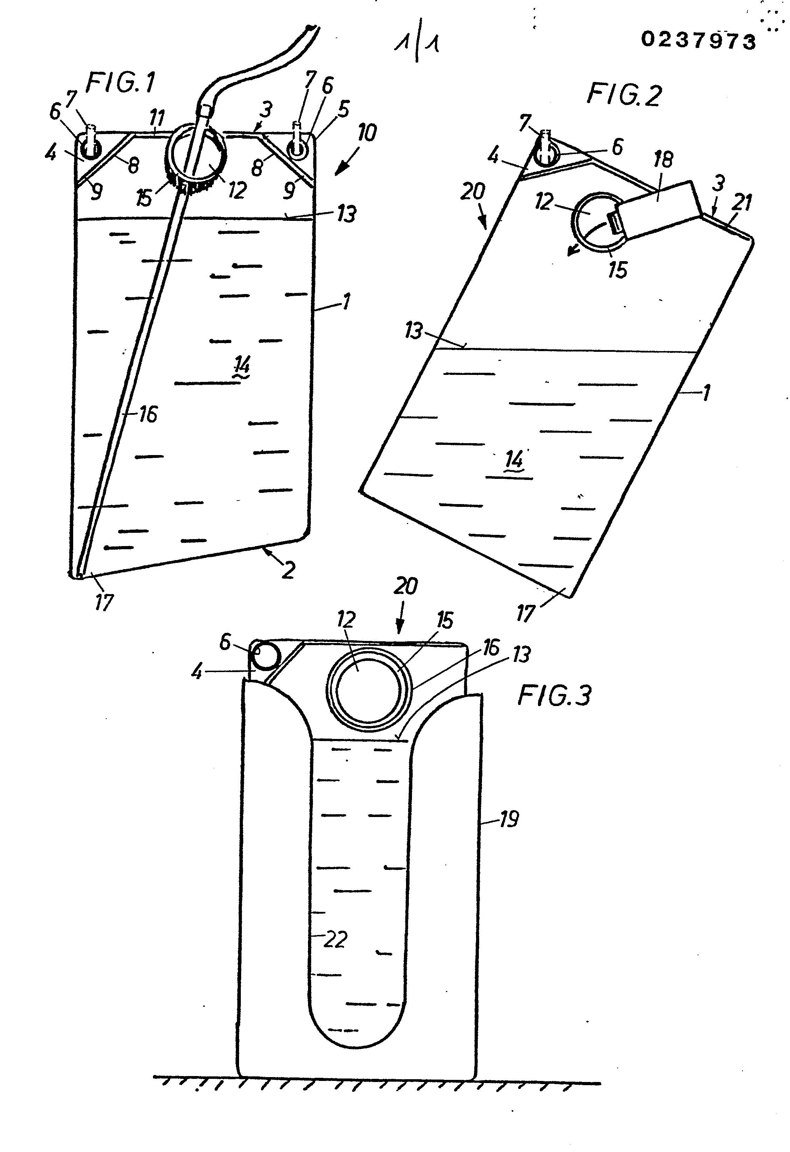

Der in Fig. 1 als Ganzes mit 10 bezeichnete Kunststoffbeutel ist ein Schlauchbeutel aus Polyäthylen oder einem ähnlichen thermoplastischen Folienmaterial, wobei sich die Längsrichtung des Schlauchabschnitts 1 gemäß Fig. 1 von oben nach unten erstreckt. Der untere Rand 2 ist gerade und bildet in dem Ausführungsbeispiel mit der Längsrichtung des Schlauchabschnitts 1 keinen rechten Winkel, sondern verläuft schräg, d.h. unter einem in dem Ausführungsbeispiel von der Querrichtung um etwa 10° abweichenden Winkel. Der obere Rand 3 verläuft bei flachgelegtem Kunststoffbeutel 10 etwa senkrecht zur Schlauchachse. In den beiden oberen Ecken sind etwa dreieckige Verstärkungsstücke 4,5 vorgesehen, die die Ösen 6 bilden, an denen der Kunststoffbeutel 10 an gestrichelt angedeuteten, in gleicher Höhe befindlichen Haken 7 so aufhängbar ist, daß er gerade hängt.

Die Verstärkungsstücke 4 sind flache Kunststoffplättchen von rechtwinklig-dreieckiger Gestalt, wobei die Schenkel des rechten Winkels bei flachgelegtem Schlauchabschnitt 1 in dessen parallel zur Schlauchrichtung gelegene Ränder bzw. in den quer verlaufenden oberen Rand 3 übergehen und wobei die Hypotenuse 8 dem Schlauchabschnitt 1 zugewandt und längs einer Schweißnaht 9 mit dem Material des Schlauchabschnitts 1 verbunden ist. Am oberen Rand 3 erstreckt sich zwischen den Verstärkungsstücken 4,5 eine Rippe 11, die durch ein separates Kunststoffteil oder aber auch durch eine entsprechend dicke Schweißnaht ausgebildet sein kann, die den Schlauchabschnitt 1 oben verschließt.

Die Entnahme- und Einfüllöffnung 12 ist nahe dem oberen Rand gelegen, so daß sie in der aus Fig. 1 ersichtlichen Weise stets oberhalb des Füllniveaus 13
des in dem Kunststoffbeutel 10 befindlichen Konzentrats
14 gelegen ist. Die Entnahme- und Einfüllöffnung 12 ist
von einem üblichen Verschlußring 15 umgeben, der mit der
Wandung des Schlauchabschnitts 1 verschweißt und im
Transportzustand von einem aufreißbaren, aufschraubbaren
oder aufschnappbaren nicht dargestellten Deckel überdeckt ist. In dem dargestellten Transportzustand ist die
Entnahme- und Einfüllöffnung 12 offen, so daß das Ansaugrohr 16 einer Konzentratpumpe bis an die tiefste Stelle
17 am unteren Rand 2 hindurchgeführt werden kann.

Bei dem Kunststoffbeutel 20 der Fig. 2 sind funktionell entsprechende Teile mit gleichen Bezugszahlen gekennzeichnet.

Im Gegensatz zu dem Kunststoffbeutel 10 weist der
Kunststoffbeutel 20 nur ein Verstärkungsstück 4 an der
linken oberen Ecke auf, so daß der Kunststoffbeutel 20
im Betrieb in der aus Fig. 2 ersichtlichen Weise schräg
hängt, so daß sich eine als Spitze ausgebildete tiefste
Stelle 17 von selbst einstellt, auch wenn der Kunststoffbeutel 20 in dem Ausführungsbeispiel am unteren Ende
rechtwinklig zur Längsrichtung des Schlauchabschnitts 1
begrenzt ist.

Von dem Verstärkungsstück 4 geht bei dem Kunststoffbeutel 20 eine Rippe 21 aus, die sich bei flachgelegtem
Schlauchabschnitt 1 quer zur Längsrichtung des Schlauchabschnitts 1 erstreckt. Das thermoplastische Folienmaterial
des Schlauchabschnitts 1 ist längs der Rippe 21 zugeschweißt.

Der Kunststoffbeutel 20 ist in Fig. 2 in der Phase
dargestellt, in welcher gerade aus einem Behälter 18 ein

- 6 -

zusätzliches Reagens dem Konzentrat 14 zudosiert wird.
Dies ist leicht möglich, weil sich die Entnahme- und Einfüllöffnung 12 in der Nähe der Öse 6 und somit oberhalb
des Füllniveaus 13 befindet, so daß kein Konzentrat aus
der Entnahme- und Einfüllöffnung 12 austreten kann.

Wenn die Zudosierung erfolgt und das zudosierte
Reagens mit dem Konzentrat 14 vermischt ist, wird wie in
Fig. 1 das Ansaugrohr 16 bis zu der tiefsten Stelle 17
eingeführt.

In Fig. 3 ist eine Aufbewahrungsphase wiedergegeben, in welcher der Kunststoffbeutel 20 in einem im wesentlichen zylindrischen Stützbehälter 19 eingesetzt ist,
so daß der Kunststoffbeutel 20 nicht umkippen kann, obwohl er nicht an der Öse 6 aufgehängt ist. Der Stützbehälter 19 weist zumindest auf einer Seite eine tiefgezogene Ausnehmung 22 auf, durch die das Füllniveau 13 des
Kunststoffbeutels 20 beobachtbar ist. Der Stützbehälter
19 kann dazu dienen, den Kunststoffbeutel 20 vorübergehend aufrecht zu halten, wenn ein Aufhängen an der Öse 6
nocht nicht möglich ist, aber auch dazu, ihn anstelle
des Aufhängens während der Entleerung aufrecht zu halten,
falls ein Aufhängen im Einzelfall nicht möglich ist.

In der Phase nach Fig. 3 ist die Entnahme- und Einfüllöffnung 12 noch durch einen mit dem Verschlußring 15
dichtend zusammenwirkenden Deckel 16 verschlossen.

Wesentlich ist für die Kunststoffbeutel 10,20, daß
sich die Entnahme- und Einfüllöffnung 12 nahe den Ösen 6
für die Aufhängung und somit über dem Füllniveau 13 befindet.

Es versteht sich, daß die Kunststoffbeutel in gefülltem Zustand tatsächlich eine bauchige Gestalt aufweisen, die in der schematischen Wiedergabe der Zeichnung
nicht zur Geltung kommt.

PATENTANWÄLTE
DIPL.-ING. WALTER KUBORN
DIPL.-PHYS. DR. PETER PALGEN
ZUGELASSEN BEIM EUROPÄISCHEN PATENTAMT
4000 DÜSSELDORF
MULVANYSTRASSE 2 · TELEFON 632727
TELEGRAMME: KUPAL
KREISSPARKASSE DÜSSELDORF NR. 1014463
DEUTSCHE BANK AG, DÜSSELDORF 2919207
POSTSCHECK-KONTO: KÖLN 115211-504

Dr. med. Ulrich Saueressig

in 5600 Wuppertal 1


Patentansprüche


1. Kunststoffbeutel zur Bevorratung von Dialysekonzentrat mit einer verschließbaren Entnahme- und Einfüllöffnung sowie mindestens einer Öse zum Aufhängen des Beutels, dadurch gekennzeichnet, daß die Entnahme- und Einfüllöffnung (12) in der Nähe der Öse (6) vorgesehen ist.

2. Kunststoffbeutel nach Anspruch 1, dadurch gekennzeichnet, daß der Kunststoffbeutel (10,20) durch einen Schlauchabschnitt (1) gebildet und an einem den oberen Rand (3) bildenden Ende rechtwinklig zur Schlauchlängsrichtung begrenzt ist und die Entnahme- und Einfüllöffnung (12) sowie die Öse (6) nahe diesem Ende angeordnet sind.

3. Kunststoffbeutel nach Anspruch 1 oder 2, dadurch gekennzeichnet, daß an dem Schlauchabschnitt (1) im Bereich der Öse (6) ein Verstärkungsstück (4,5) vorgesehen ist, welches die Öse (6) enthält.

4. Kunststoffbeutel nach Anspruch 3, dadurch gekennzeichnet, daß von dem Verstärkungsstück (4,5) eine Rippe (11,21) zur Verstärkung des oberen Randes (3) des Kunststoffbeutels (10,20) ausgeht.

0237973

- 2 -

5. Kunststoffbeutel nach Anspruch 3 oder 4, dadurch gekennzeichnet, daß das Verstärkungsstück (4,5) als dreieckiges flaches Plättchen ausgebildet ist, welches eine obere Ecke des Kunststoffbeutels (10,20) bildet.

6. Kunststoffbeutel nach einem der Ansprüche 1 bis 5, dadurch gekennzeichnet, daß der flachgelegte Kunststoffbeutel (10) an dem der Entnahme- und Einfüllöffnung (12) und der Öse (6) gegenüberliegenden Rand (2) schräg begrenzt ist.

7. Kunststoffbeutel nach einem der Ansprüche 1 bis 6, dadurch gekennzeichnet, daß ihm ein becherartiger Stützbehälter (19) zugeordnet ist, in den der Kunststoffbeutel (10,20) einsetzbar ist und der an mindestens einer Seite eine tief herabgezogene Ausnehmung zwecks Beobachtung des Füllniveaus (13) aufweist.

0237973

FIG.1

FIG.2

FIG.3

# EUROPÄISCHER RECHERCHENBERICHT

Europäisches Patentamt

## EINSCHLÄGIGE DOKUMENTE

EP 87103641.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | DE - B2 - 2 312 371 (UNION CARBIDE) | 1,2 | A 61 M 5/00 |
| A | * Gesamt * | 3,4 | A 61 M 1/14 |
| | -- | | A 61 J 1/00 |
| X | FR - A - 2 098 873 (LAB. LABAZ) | 1,2 | B 65 D 85/00 |
| A | * Gesamt; insbesondere Fig. 1,7; Seite 5, Zeile 38 - Seite 6, Zeile 2; Seite 8, Zeilen 1-5 * | 3,4 | |
| | -- | | |
| X | US - A - 3 304 977 (B.G.HAMMONS) | 1 | |
| A | * Gesamt; insbesondere Fig. 1,5; Spalte 2, Zeilen 47-59 * | 2,3,5 | |
| | -- | | |
| X | FR - A - 2 104 036 (B.H.CHRISTENSEN) | 1 | |
| A | * Gesamt; insbesondere Seite 2, Zeilen 21-31 * | 2-4 | |
| | ---- | | |

RECHERCHIERTE SACHGEBIETE (Int. Cl.4)

A 61 J 1/00
A 61 M 1/00
A 61 M 5/00
B 65 D 30/00
B 65 D 33/00
B 65 D 85/00

Der vorliegende Recherchenbericht wurde für alle Patentansprüche erstellt.

| Recherchenort | Abschlußdatum der Recherche | Prüfer |
|---|---|---|
| WIEN | 16-07-1987 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82